# EUROPÄISCHE PATENTSCHRIFT

(11) **EP 0 660 736 B1**
(45) Veröffentlichungstag und Bekanntmachung des Hinweises auf die Patenterteilung: **05.03.1997**
(21) Anmeldenummer: 93919017.9
(22) Anmeldetag: 16.09.1993
(51) Int. Cl.: A61N 1/365

(54) **SCHALTUNG ZUR MESSUNG DER IMPEDANZ IM HERZEN**
CIRCUIT FOR MEASURING IMPEDANCE IN THE HEART
CIRCUIT POUR MESURER L'IMPEDANCE DU COEUR

(30) Priorität: 17.09.1992 DE 4231602
(43) Veröffentlichungstag der Anmeldung: 05.07.1995
(73) Patentinhaber: BIOTRONIK Mess- und Therapiegeräte GmbH & Co Ingenieurbüro Berlin, D-12359 Berlin (DE)
(72) Erfinder: SCHALDACH, Max, D-91054 Erlangen (DE)
(74) Vertreter: Christiansen, Henning, Dipl.-Ing.
(86) Internationale Anmeldenummer: DE9300888
(87) Internationale Veröffentlichungsnummer: WO9406512

(56) Entgegenhaltungen:
- EP-A- 0 134 908
- EP-A- 0 151 689
- US-A- 3 871 359
- US-A- 4 702 253
- BIOMEDIZINISCHE TECHNIK, Bd.32, September 1987, BERLIN DE Seiten 41 - 42 G. BOHEIM ET. AL. 'intrkardiale Impedanzmessung zur Regelung frequenzadaptiver Schrittmachersysteme'

## Beschreibung

Die Erfindung betrifft eine Anordnung der im Oberbegriff des Anspruchs 1 angegebenen Art.

In einigen Fällen ist es bei dem Betrieb von Herzschrittmachern, insbesondere im Zusammenhang mit einer Anpassung der Stimulationsrate an die aktuelle Belastung des Patienten, günstig, die elektrische Impedanz im Herzbereich meßtechnisch zu erfassen.

Dabei ist es beispielsweise bekannt, das Herzschlagvolumen nach Art der Impedanz-Plethysmographie, d.h. durch eine Messung der Impedanz im Herzen bestimmen. Dazu werden im einfachsten Fall zwei Elektroden ins Herz eingeführt. Fließt über diese Elektroden ein Strom bekannter Größe durch das Herzvolumen, so läßt sich über eine Spannungsmessung die Impedanz im Herzen bestimmen. In der DE-36 29 587-A1 ist eine entsprechende Vorrichtung zur Messung des Ventrikularvolumens beschrieben. Eine derartige Anordnung ist für die Anwendung bei Herzschrittmachern nicht geeignet.

EP-A-0 151 689 beschreibt eine Vorrichtung zur Messung des Ventrikularvolumens bei Herzschrittmachern, bei der langsame Veränderungen der gemessenen Impedanz als Folge von sich ändernden Meßbedingungen ausgeglichen werden.

Aus der EP-A-0 140 472 ist ein Herzschrittmacher bekannt, bei dem über an der Schrittmacherelektrode angebrachte weitere Elektroden die Impedanz im Herzen gemesssen wird und mit dem daraus bestimmten Schlagvolumen die Stimulationsrate gesteuert wird. Es ist dabei eine Folge von Messungen vorgesehen, aus deren Mittelwert sich das Schlagvolumen berechnet.

Aus der US-A-3 871 359 ist ferner ein System bekannt, bei dem gleichzeitig die Impedanz an zwei verschiedenen Stellen im Herzen mittels einer Konstant-Wechselstromquelle gemessen wird. Damit können gleichgerichtete Störanteile durch Subtraktion der beiden Meßsignale teilweise kompensiert werden, wobei beiden Meßpunkten der gleiche Konstantstrom zugeführt wird.

Der Erfindung liegt die Aufgabe zugrunde, eine Anordnung der eingangs genannten Gattung anzugeben, die insbesondere für den Einsatz bei Herzschrittmachern geeignet ist.

Diese Aufgabe wird mit den kennzeichnenden Merkmalen des Anspruchs 1 gelöst.

Die Erfindung geht von der Erkenntnis aus, daß es bei Impedanzmessungen im Herzen, die letztendlich immer auf kombinierte Strom / Spannungsmessungen hinauslaufen, wichtig ist, die dort auftretenden Störspannungen, zu denen unter anderem eine vom Stimulationsimpuls herrührende Polarisationsspannung gehören kann, möglichst zu eliminieren. Dies muß mit einem wegen der begrenzten Batterieladung des Schrittmachers möglichst geringen Energieaufwand geschehen.

Bei der erfindungsgemäßen Schaltung ist die Schrittmacherelektrode mit einer in ihrer Stromrichtung umschaltbaren und in ihrer Stromstärke regelbaren Stromquelle verbunden. Die an der Schrittmacherelektrode anliegende Spannung wird dabei von mindestens einer Vorrichtung zur Spannungsmessung gemessen.

Die Messung erfolgt insbesondere außerhalb der Zeiten der Stimulationsimpulse oder der Zeiten, die für eine Erfassung der vom Herzen abgegebenen Signale zur Verfügung stehen müssen.

Ist eine nicht vom Stromfluß herrührende Spannung an der Schrittmacherelektrode vorhanden, so steuert diese einen entsprechenden Beitrag zur gemessenen Spannung bei, d.h. diese Spannungsmessung ist fehlerbehaftet. Dieser Meßfehler wird eleminiert, indem die Stromrichtung durch entsprechende Schaltungsmittel umgekehrt und die an der Schrittmacherlektrode anliegende Spannung erneut gemessen wird. Bei dieser Messung liefert die nicht vom Stromfluß herrührende Spannung den gleichen Beitrag wie zur ersten Messung. Bei der Subtraktion der beiden gemessenen Spannungen heben sich diese Beiträge gegenseitig auf, während sich die nicht fehlerbehafteten Anteile wegen ihres durch die Stromumkehr entgegengesetzten Vorzeichens addieren.

Die zur Messung erforderlichen Ströme müssen auf diese Weise nicht so groß gemacht werden, daß der durch sie hervorgerufene Spannungsabfall an der Impedanz wesentlich größer als die Polarisationsspannung ist. Dies führt zu einer vergrößerten Lebensdauer der Schrittmacherbatterie.

Bei einer vorteilhaften Weiterbildung der Erfindung wird die Größe des von der Stromquelle abgegeben, über die Schrittmacherelektrode fließenden Stroms in Abhängigkeit von der Differenz der im stromlosen Zustand nacheinander gemessenen Spannungen geregelt. Auf diese Weise läßt sich die Stärke des Stroms optimal dem nicht kompensierbaren Störpegel anpassen, was zu einer weiteren Entlastung der Schrittmacherbatterie führt.

Vorteilhafterweise erfolgt die Stromabgabe der Stromquelle in Form von Impulspaketen, wobei jedes Impulspaket aus zwei kurz hintereinenanderfolgenden Rechteckimpulsen besteht. Dies vereinigt mehrere Vorteile: Zum einen veringert sich der Energiebedarf der Messung wesentlich, zum anderen lassen sich so bei einer sich zeitlich ändernden Impedanz, was im Fall des schlagenden Herzens der Fall ist, deren Momentanwerte messen. Diese Werte sind, da sich die äußere, nicht vom Meßstrom hervorgerufene Spannung bei unmittelbar aufeinanderfolgenden Impulsen nur wenig ändert, genauer als durch lange Mittelung erhaltene.

Bei einer anderen vorteilhaften Weiterbildung der Erfindung erfolgen die Einzelmessungen, d.h. die Abgabe der Impulspakete, in gegenüber dem zeitlichen Abstand zwischen den Einzelimpulsen großen Zeitabständen. Diese Beschränkung auf wenige Impulspakete ist in Zusammenhang mit der in Abhängigkeit vom Störpegel regelbaren Stromstärke besonders vorteilhaft. Der Störabstand erhöht sich nämlich linear mit der Zunahme des Stroms, d.h. mit dem Stromverbrauch, während bei einer statistischen Ausmittelung der Störabstand dagegen nur mit der Wurzel aus der Anzahl der Messungen und damit nur mit der Wurzel des Stromverbrauchs zunimmt. Bei implantierbaren Herzschrittmachern ist dies von großer Bedeutung, da ein Batteriewechsel immer mit einer Operation verbunden ist.

Oftmals ist nicht notwendig, den Absolutwert der Impedanz im Herzen zu kennen, vielmehr sind Informationen über deren relative Änderung ausreichend. Solche Relativmessungen sind mit wesentlich größerer Genauigkeit durchzuführen als eine Absolutmessung, vorallem wenn die zu messenden Signale sehr klein sind. In solchen Fällen können die Meßschaltungen selbst Fehler erzeugen.

Bei der erfindungsgemäßen Schaltung wird deshalb zunächst eine für die zeitliche Änderung der Impedanz im Herzen repräsentative Spannung gebildet, indem von einer zu einem bestimmten Zeitpunkt gemessenen für die Impedanz repräsentativen Spannung eine weitere zu einem späteren Zeitpunkt gemessenene Spannung subtrahiert wird, wobei diese beiden Spannungen durch Messungen bei in ihrer Impulsfolge übereinstimmenden Impulspaketen erhalten werden. Eventuell durch die Schaltung selber hinzugefügte Fehlspannungen werden durch diese Maßnahme eliminiert.

In einem weiteren Schritt, wird zu der so erhaltenen Differenzspannung eine weitere Differenzspannung addiert. Diese weitere Differenzspannung wird wie oben gewonnen, jedoch durch Spannungsmessungen an Impulspaketen mit entgegengesetzter Impulsfolge. Dadurch wird zunächst auch eine Spannung mit entgegengesetzten Vorzeichen gemessen, das durch Schaltungsmittel mit einer in ihrem Vorzeichen steuerbaren Verstärkung wieder umgedreht wird.

Vorzeichenabhängige Fehlspannungen werden jetzt durch die Summation der beiden Differenzspannungen eleminiert.

Am Ausgang der Schaltung erscheint nun eine Summe von zeitlichen Änderungen der Impedanz im Herzen, aus der sich durch Mittelwertsbildung die durchschnittliche zeitliche Änderung ermitteln läßt.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein Blockschaltbild des Ausführungsbeispiels der Erfindung
Figur 2 ein Schaltbild einer Eingangsstufe des Ausführungsbeispiels nach Figur 1
Figur 3 einen Teil des Ausführungsbeispiels nach Figur 1 und 2
Figur 4 ein Blockschaltbild eines Teils des Ausführungsbeispiels der Erfindung gemäß Figuren 1 bis 3
Figur 5 ein Schaltbild einer Stromquelle nach Figur 1

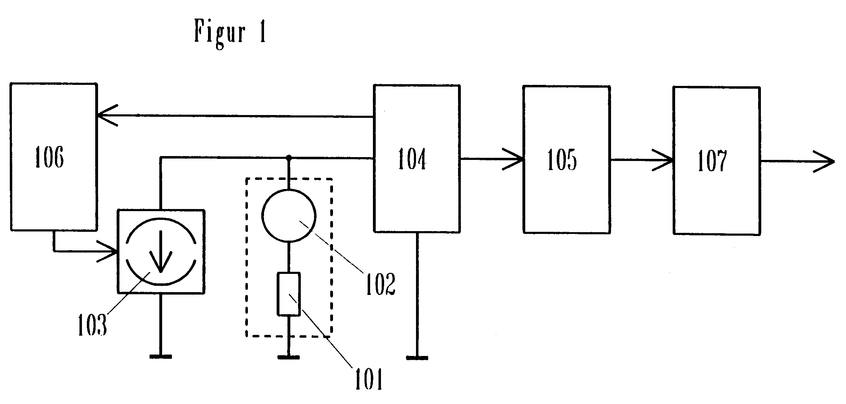

Figur 1 zeigt den grundsätzlichen Aufbau der erfindungsgemäßen Schaltung in einem Blockschaltbild. Die zu erfassende Impedanz im Herzen ist als Widerstand 101 mit einer in Reihe geschalteten Spannungsquelle 102 dargestellt. Die Spannungsquelle 102 symbolisiert die vom Schrittmacherimpuls herrührende Polarisationsspannung und andere Störspannungen. Die Impedanz wird von einer Stromquelle 103 mit einem Strom beaufschlagt. Die an der Impedanz anliegende Spannung wird mit einem Abtastbaustein 104 erfaßt. Dessen Ausgangssignal wird an einen ersten Differenzenverstärker 105 weitergegeben, dessen Ausgangsignal von der Differenz zweier Spannungen bestimmt wird, die zu verschiedenen Zeiten durch den Abtastbaustein 104 gemessen wurden. Desweiteren ist der Abtastbaustein 104 mit einer Regelung 106 verbunden, die die Höhe des von der Stromquelle 103 abgegebenen Stroms in Abhängigkeit vom aktuellen Störspannungspegel regelt.

Das vom Differenzverstärker 105 kommende Signal wird an einen zweiten Differenzverstärker 107 weitergeleitet. Dieser bildet die Differenz zweier Differenzspannungen unterschiedlichen Vorzeichens. Das Ausgangssignal des Differenzverstärkers 107 ist das Ausgangssignal der Schaltung.

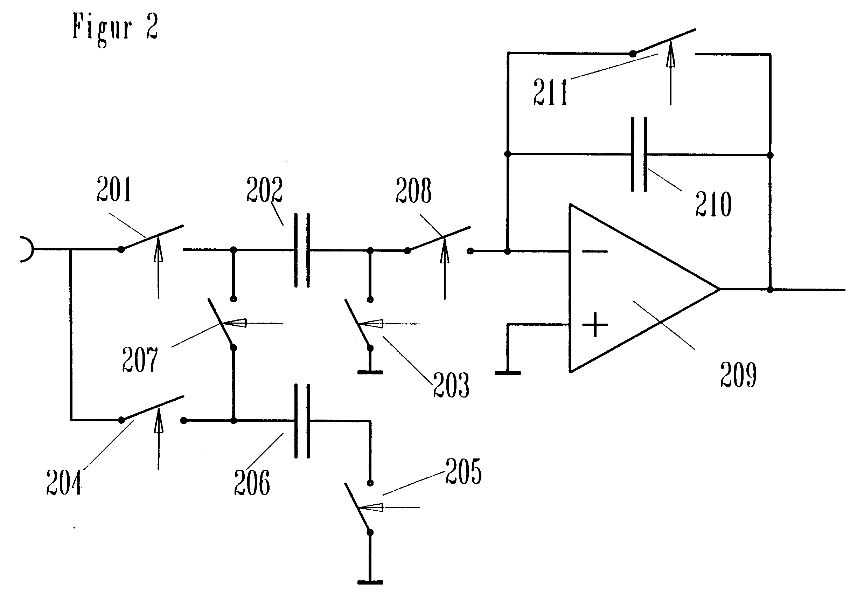

Figur 2 zeigt ein Schaltbild einer Eingangsstufe des Abtastbausteins 104. Die an der Impedanz im Herzen anliegende Spannung liegt über einen steuerbaren Schalter 201 an einem Kondensator 202 an, der über einen weiteren steuerbaren Schalter 203 mit Masse verbunden ist. Der Kondensator wird durch die an der Impedanz anliegende Spannung geladen.

Nach einer gewissen Zeit öffnen die Schalter 201 und 203, worauf zwei weitere steuerbare Schalter 204 und 205 schließen und ein Kodensator 206 geladen wird.

In einem weiteren Schritt öffnet der Schalter 204. Danach wird ein steuerbarer Schalter 207 zusammen mit einem weiteren steuerbaren Schalter 208 geschlossen. Die Differenz der Spannungen der Kondensatoren 206 und 202 liegt nun am Eingang eines Operationsverstärkers 209. In dessen Gegenkopplungszweig liegen ein Kondensator 210 und ein weiterer steuerbarer Schalter 211.

Der so als Integrator beschaltete Operationsverstärker kompensiert die durch unvermeidliche Leckströme der Kondensatoren 202 und 206 auftretenden Verluste, so daß am Ausgang der Eingangsstufe ein der Differenz der zu unterschiedlichen Zeiten an der Impedanz anliegenden Spannungen proportionales Signal erscheint.

Werden diese Spannungen bei entgegengesetzten Strömen durch die Impedanz gemessen, so kompensieren sich die in beiden Fällen mit gleichen Vorzeichen auftretenden von den Strömen unabhängigen Störspannungen gegenseitig.

Die zur Steuerung der Schalter notwendigen Impulse werden aus dem im Schrittmacher vorhandenen Taktgeber gewonnen.

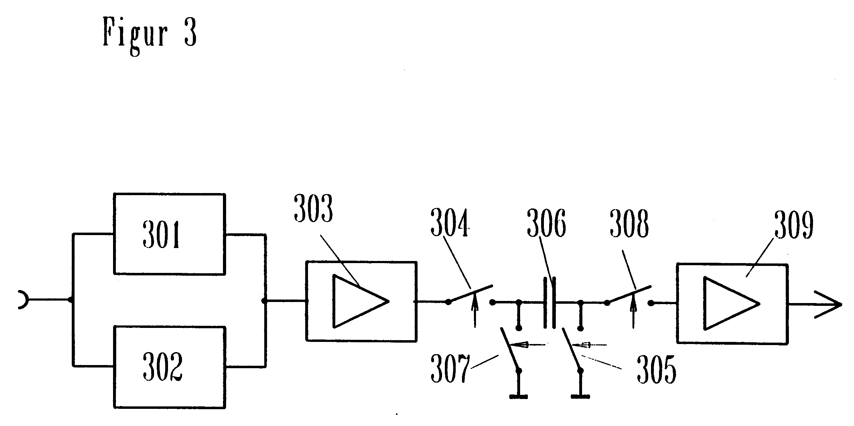

Figur 3 zeigt einen Teil der Schaltung. Am Eingang befinden sich zwei Schaltungsblöcke 301 und 302. Dabei stellt jeder dieser Blöcke eine Eingangsstufe der oben beschriebenen Art dar. Diese liefern zu unterschiedlichen Zeitpunkten für die Impedanz im Herzen repräsentative Ausgangsspannungen, die einem Verstärker 303 zugeführt werden.

Mit dem verstärkten Signal der Eingangsstufe 301 wird über die Schalter 304 und 305 ein Kondensator 306 geladen. Mit Hilfe weiterer Schalter 307 und 308 liegt dann die Spannung des Kondensators 306 mit umgekehrten Vorzeichen am Eingang eines weiteren Verstärkers 309. Das verstärkte Signal der Eingangstufe 302 wiederum wird über die Schalter 304 und 308 über den Kondensator 306 vorzeichengleich dem Verstärker 309 zugeführt, so daß an dessen Ausgang die Differenz der verstärkten Spannungen der Eingangsstufen 301 und 302 erscheint.

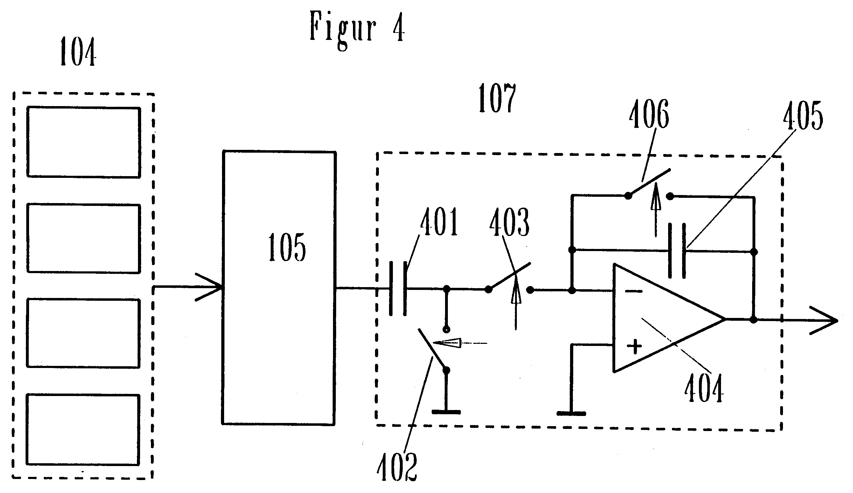

Figur 4 zeigt ein Blockschaltbild der Schaltung mit der Abtasteinheit 104, den Differenzverstärker 105 und der detailliert dargestellten Schaltungsstufe 107. Die Differenzspannung zweier Abtasteinheiten wird an einen Kondensator 401 gelegt. Dabei ist ein Schalter 402 geschlossen. Mit Hilfe eines weiteren Schalters 403 dient die Spannung des Kondensators 401 als Referenz für eine vorzeichenentgegengesetzte Differenzspannung zweier weiterer Eingangstufen der Abtasteinheit. Am Ausgang eines Operationsverstärkers 404, in dessen Gegenkopplungszweig ein Kondensator 405 und ein Schalter 406 liegen, erscheint die Summe der beiden Differenzspannungen.

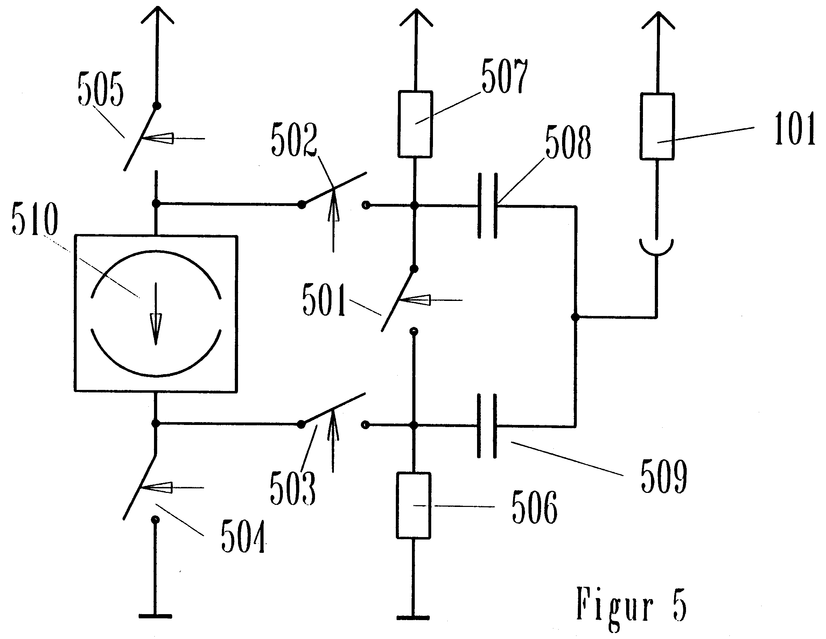

Figur 5 zeigt das Schaltbild einer Stromquelle des Ausführungsbeispiels. Zur Verdeutlichung des Stromflusses ist auch die zu messende Impedanz 101 eingezeichnet.

Im Ruhezustand, d.h. ohne Stromabgabe über die Impedanz 101, ist ein Schalter 501 geschlossen. Weitere Schalter 502, 503, 504 und 505 sind geöffnet. Zwei Widerstände 506 und 507 halten zwei Kondensatoren 508 und 509 auf halber Betriebsspannung.

Um einen positiven Stromimpuls über die Impedanz 101 abzugeben, wird der Schalter 501 geöffnet und die Schalter 503 und 505 werden geschlossen. Die Spannung des Kondensators 509 liegt nun in Reihe mit der positiven Betriebsspannung, so daß sich der Kondensator über die Impedanz entlädt. Die Größe des fließenden Stroms wird dabei von einer Stromquelle 510 bestimmt.

Bei einem negativen Stromimpuls werden die Schalter 502 und 504 geschlossen, so daß der Kondensator 508 über die Impedanz 101 geladen wird. Da die Stromquelle 510 wiederum in Reihe mit der Impedanz 101 und dem Kondensator 508 liegt, bestimmt sie auch hier den fließenden Strom.

## Patentansprüche

1. Schaltung zur Erfassung der Impedanz im Herzen über eine in der Herzkammer angeordnete Schrittmacherelektrode mit einer mit der Schrittmacherelektrode verbundenen, durch Schaltungsmittel in ihrer Stromrichtung umschaltbaren Stromquelle und mindestens einer Vorrichtung zur Messung der an der Schrittmacherelektrode anliegenden Spannung,
**dadurch gekennzeichnet,**
daß ein für die Impedanz im Herzen repräsentativer Wert aus der Differenz der an der Schrittmacherelektrode zu zwei unterschiedlichen Meßzeitpunkten anliegenden Spannungen ermittelt wird, wobei der Schrittmacherelektrode zu diesen Zeitpunkten jeweils betragsmäßig gleiche, jedoch entgegengesetzt gerichtete Ströme aufgeprägt werden.

2. Schaltung nach Anspruch 1, **dadurch gekennzeichnet,** daß die Größe des von der Stromquelle abgegebenen, über die Schrittmacherelektrode fließenden Stroms in Abhängigkeit von der Größe der Differenz der zu unterschiedlichen Zeitpunkten im stromlosen Zustand an der Schrittmacherelektrode anliegenden Spannungen eingestellt wird.

3. Schaltung nach einem der Ansprüche 1 bis 2, **dadurch gekennzeichnet,** daß die Stromquelle die Form von Impulspaketen aufweist, wobei jedes Impulspaket aus zwei kurz aufeinanderfolgenden Rechteckimpulsen entgegengesetzter Polarität besteht.

4. Schaltung nach einem der Ansprüche 1 bis 3, **dadurch gekennzeichnet,** daß der zeitliche Abstand zwischen den Impulspaketen groß gegenüber dem zeitlichen Abstand zwischen den einzelnen Rechteckimpulsen innerhalb eines Impulspakets ist.

5. Schaltung nach einem der Ansprüche 1 bis 4, **dadurch gekennzeichnet,** daß die Impulsfolge aufeinanderfolgende Impulspakete entgegengesetzter Polarität enthält.

6. Schaltung nach einem der Ansprüche 1 bis 5, **dadurch gekennzeichnet,** daß das Vorzeichen der für die Impedanz repräsentativen Spannung bei allen Impulspaketen durch Schaltungsmittel mit einer in ihrem Vorzeichen steuerbaren Verstärkung unverändert bleibt.

7. Schaltung nach einem der Ansprüche 1 bis 6, **dadurch gekennzeichnet,** daß eine für die zeitliche Änderung der Impedanz im Herzen repräsentative Differenzspannung dadurch gebildet wird, indem von der zu einem bestimmten Zeitpunkt gemessenen, für die Impedanz im Herzen repräsentativen Spannung eine weitere zu einem späteren Zeitpunkt gemessene, für die Impedanz im Herzen repräsentative Spannung subtrahiert wird, wobei diese beiden Spannungen durch Messungen bei in ihrer Impulsfolge übereinstimmenden Impulspaketen erhalten werden.

8. Schaltung nach einem der Ansprüche 1 bis 7, **dadurch gekennzeichnet,** daß die Summe aus zwei für die zeitliche Änderung repräsentativen Differenzspannungen gebildet wird, wobei diese Differenzspannungen jeweils durch Messungen bei sich in ihrer Impulsfolge unterscheidenden Impulspaketpaaren erhalten werden.

## Claims

1. A circuit for detecting the impedance in the heart via a pacemaker electrode arranged in the heart chamber comprising a current source which is connected to the pacemaker electrode and which can be switched over in its current direction by switching means and comprising at least one device for measuring the voltage connected to the pacemaker electrode, characterised in that a value representative of the impedance in the heart is determined from the difference between the voltages connected to the pacemaker electrode at two different measurement times, where at these times currents which in each case are equal in amount but are oppositely directed are impressed upon the pacemaker electrode.

2. A circuit according to Claim 1, characterised in that the magnitude of the current emitted from the current source and flowing across the pacemaker electrode is set as a function of the magnitude of the difference between the voltages connected to the pacemaker electrode at different times in the current-free state.

3. A circuit according to one of Claims 1 to 2, characterised in that the current source has the form of pulse packets, where each pulse packet comprises two closely consecutive rectangular pulses of opposite polarity.

4. A circuit according to one of Claims 1 to 3, characterised in that the interval of time between the pulse packets is large compared to the interval of time between the individual rectangular pulses within a pulse packet.

5. A circuit according to one of Claims 1 to 4, characterised in that the pulse sequence comprises consecutive pulse packets of opposite polarity.

6. A circuit according to one of Claims 1 to 5, characterised in that the sign of the voltage representative of the impedance remains unchanged in the case of all the pulse packets by means of switching means with an amplification which is controllable in respect of its sign.

7. A circuit according to one of Claims 1 to 6, characterised in that a difference voltage representative of the time change of the impedance in the heart is formed in that from the voltage representative of the impedance in the heart and measured at a specified time, a further voltage representative of the impedance in the heart and measured at a later time is subtracted, where these two voltages are obtained by measurements of pulse packets which correspond to one another in their pulse sequence.

8. A circuit according to one of Claims 1 to 7, characterised in that the sum of two difference voltages representative of the time change is formed, where these difference voltages are in each case obtained by measurements of pairs of pulse packets which differ in their pulse sequence.

## Revendications

1. Circuit pour saisir l'impédance dans le coeur au moyen d'une électrode de stimulateur cardiaque disposée dans le ventricule, comportant une source de courant reliée à l'électrode du stimulateur cardiaque et dont un moyen de commutation peut commuter le sens de son courant et d'au moins un dispositif pour mesurer la tension qui apparaît à l'électrode du stimulateur cardiaque,
caractérisé
par le fait que l'on détermine une valeur représentative pour l'impédance dans le coeur à partir de la différence des tensions apparaissant à l'électrode du stimulateur cardiaque à deux instants de mesure différents, étant précisé que ce sont des courants respectivement identiques en valeur absolue, mais orientés en sens opposé, qui sont imprimés à l'électrode du stimulateur cardiaque à ces instants.

2. Circuit selon la revendication 1, caractérisé par le fait que l'on règle la valeur du courant fourni par la source de courant et passant par l'électrode du stimulateur cardiaque en fonction de la valeur de différence des tensions apparaissant à l'électrode du stimulateur cardiaque à des instants différents, en l'absence de courant.

3. Circuit selon l'une des revendications 1 à 2, caractérisé par le fait que la source de courant présente la forme de paquets d'impulsions, chaque paquet d'impulsions étant constitué de deux brèves impulsions rectangulaires successives de polarité opposée.

4. Circuit selon l'une des revendications 1 à 3, caractérisé par le fait que l'intervalle de temps entre les paquets d'impulsions est grand par rapport à l'intervalle de temps entre les différentes impulsions rectangulaires à l'intérieur d'un paquet d'impulsions.

5. Circuit selon l'une des revendications 1 à 4, caractérisé par le fait que la suite d'impulsions contient des paquets d'impulsions successifs de polarité opposée.

6. Circuit selon l'une des revendications 1 à 5, caractérisé par le fait que le signe de la tension représentative de l'impédance reste inchangé pour tous les paquets d'impulsions grâce à des moyens de commutation dont on peut commander le signe de l'amplification.

7. Circuit selon l'une des revendications 1 à 6, caractérisé par le fait que l'on forme une différence de tension représentative de la modification, de l'impédance dans le coeur en fonction du temps en soustrayant, de la tension mesurée à un instant déterminé, représentative de l'impédance dans le coeur, une autre tension mesurée à un instant ultérieur, représentative de l'impédance dans le coeur, ces deux tensions étant obtenues par des mesures faites dans le cas de paquets d'impulsions dont la série d'impulsions coïncide.

8. Circuit selon l'une des revendications 1 à 7, caractérisé par le fait que l'on forme la somme de deux différences de tension représentatives de la modification en fonction du temps, ces différences de tension étant chacune obtenues par des mesures faites dans le cas de paires de paquets d'impulsions de suite d'impulsions différente.
